# EUROPÄISCHE PATENTSCHRIFT

(11) **EP 2 976 091 B1**
(45) Veröffentlichungstag und Bekanntmachung des Hinweises auf die Patenterteilung: **07.06.2023**
(21) Anmeldenummer: 14719638.0
(22) Anmeldetag: 19.03.2014
(51) Int. Cl.: A61K 33/42, A61K 31/409, A61K 36/82, A61K 36/87, A61K 36/704, A61K 36/185, A61K 36/38, A61K 36/48, A61P 39/06

(54) **ANTIOXIDATIV WIRKSAME ZUSAMMENSETZUNG UND DEREN VERWENDUNG**
ANTIOXIDATIVELY ACTIVE COMPOSITION AND THE USE THEREOF
COMPOSITION À ACTION ANTIOXYDANTE ET UTILISATION DE LADITE COMPOSITION

(30) Priorität: 21.03.2013 DE 102013205049
(43) Veröffentlichungstag der Anmeldung: 27.01.2016
(73) Patentinhaber: LR Health & Beauty Systems GmbH, 59227 Ahlen (DE)
(72) Erfinder: LARSEN-VEFRING, Sabine, 14089 Berlin (DE); LENDZIAN, Christian, 59302 Oelde (DE); GASSEN, Monika, 53844 Troisdorf (DE)
(74) Vertreter: Maikowski & Ninnemann Patentanwälte Partnerschaft mbB
(86) Internationale Anmeldenummer: PCT/EP2014/055483
(87) Internationale Veröffentlichungsnummer: WO 2014/147112

(56) Entgegenhaltungen:
- WO-A1-01/62265
- WO-A1-2009/059205
- WO-A1-2013/093863
- US-A1- 2008 305 096

## Beschreibung

Die vorliegende Erfindung betrifft eine antioxidativ wirksame Zusammensetzung nach Anspruch 1. **Beschreibung**

Antioxidantien haben große physiologische Bedeutung durch ihre Wirkung als Radikalfänger. Sie inaktivieren im Organismus reaktive Sauerstoffspezies (ROS), deren Vorkommen im Übermaß zu oxidativem Stress führt. Oxidativer Stress gilt als mitverantwortlich für den Alterungsprozess und wird in Zusammenhang mit der Entstehung einer Reihe von Krankheiten gebracht.

Oxidation und Reduktion stellen wichtige biochemische Reaktionen in den menschlichen Zellen dar. Als Zwischenprodukte fallen instabile, hochreaktive und kurzlebige Substanzen an, z.B. Sauerstoffradikale. Unter freien Radikalen versteht man Atome, Ionen oder Moleküle mit einem oder mehreren ungepaarten Elektron in der äußeren Schale. Diese ungepaarten Elektronen haben das Bestreben, sich mit einem weiteren Elektron zu paaren und weisen daher eine hohe Reaktivität auf. Unter dem Begriff "reaktive Sauerstoffspezies" (ROS: reactive Oxygen Species) werden die Sauerstoffmetaboliten und Sauerstoffradikale zusammengefasst. Einerseits haben die ROS spezifische physiologische Funktionen. Sie töten Keime intrazellulär ab, sind regulativ im Stoffwechsel aktiv, kontrollieren u.a. Enzymreaktionen und regulieren die Genexpression über redoxsensitive Transkriptionsfaktoren. Sie können aber auch toxische Wirkung besitzen, die zu subzellulären und zellulären Schäden führen können.

Der Organismus verfügt über ein komplexes antioxidatives Schutzsystem in Form eines ausgewogenen Verhältnisses zwischen Synthese und Abbau von reaktiven Sauerstoffspecies. Kommt es durch Störungen dieses Gleichgewichtes zu einer Anreicherung von ROS, können schwerwiegende Funktionsstörungen auftreten. Dieser Zustand wird oxidativer Stress genannt. Zu den Folgen des oxidativen Stresses gehören die Lipidperoxidation, die letztlich dazu führt, dass Zellen mehr Energie aufwenden müssen, um ihr Membranpotenzial zu stabilisieren, die Proteinoxidation und die Schädigung der DNA. Diese drei Vorgänge werden mitverantwortlich für den Alterungsprozess und die Lebenserwartung gemacht.

Im Organismus befinden sich endogene und exogene Faktoren, die einen effektiven Schutz vor freien Radikalen darstellen. Sie müssen die Balance zwischen notwendiger Radikalbildung und Radikal-Übermaß austarieren. Diese Verbindungen werden Antioxidantien genannt. Ein Antioxidans ist eine chemische Verbindung, die eine unerwünschte Oxidation anderer Substanzen gezielt verhindert. Antioxidantien haben große physiologische Bedeutung durch ihre Wirkung als Radikalfänger. Sie inaktivieren im Organismus reaktive Sauerstoffspezies (ROS), deren Vorkommen im Übermaß zu oxidativem Stress führt.

Lebensstil und berufliche Gegebenheiten beeinflussen die exogene Belastung durch freie Radikale. Zu den Personengruppen mit erhöhtem oxidativen Stress zählen insbesondere Raucher, Personen mit chronischer Medikamenten-Einnahme, Leistungssportler, Innenstadtbewohner, regelmäßige, starke Sonnenlicht-/UV-Exposition, Chronisch Kranke (rheumatische Erkrankungen, Diabetiker) und auch Flugpersonal.

So nehmen Raucher beim Inhalieren von Rauch große Mengen an freien Radikalen auf. Durch Inhaltsstoffe des Zigarettenrauches kommt es zu weiterer Bildung freier Radikale im Organismus. Anhand von Studien mit Vitamin C wurde ermittelt, dass Raucher einen erhöhten Bedarf an Antioxidantien haben, eine ungünstigere Versorgung mit Antioxidantien und einen höheren metabolischen Umsatz. Der erhöhten Radikalbelastung von Rauchern steht eine ungünstige Versorgung mit Antioxidantien gegenüber.

Die Einnahme von Medikamenten bei Erkrankungen ist häufig mit erhöhter Radikalbelastung verbunden. Die Freisetzung von Sauerstoff-Radikalen kann entweder der gewünschte Wirkmechanismus sein (z.B. bei Antimalariamitteln) oder es handelt sich um eine unerwünschte Nebenwirkung. Cytostatika, Laxanzien, Paracetamol oder Antibiotika wie Chloramphenicol können durch enzymatische Arzneistoffmetabolisierung SauerstoffRadikale freisetzen. Es sind auch nicht-enzymatische Arzneistoff-Metabolisierungen bekannt, z.B. Autoxidation mit Bildung radikalischer Zwischenprodukte. Hierzu gehören u.a. Dopa und Adrenalin.

Antioxidantien werden gruppiert in natürliche (von Natur aus in Lebensmitteln), naturidentische (den natürlichen in ihrer chemischen Struktur identisch aber synthetisch hergestellt) und synthetische Antioxidantien. Sie hemmen beim Menschen widrige Effekte reaktiver Stoffwechselprodukte, werden aber auch Lebensmitteln zugesetzt, da sie in kleinen Konzentrationen in der Lage sind, die Autoxidation von leichtoxidierbaren Lebensmitteln (z.B.Fetten) zu hemmen.

Antioxidantien haben sehr unterschiedliche chemische Strukturen. Ihre Gemeinsamkeit besteht in der Fähigkeit, unerwünschte Oxidationen zu verhindern, und Radikalreaktionen zu hemmen. Reaktionspartner von Oxidationsrektionen ist immer Sauerstoff aus der Luft.. In der äußeren Elektronenhülle des Sauerstoffmoleküls befinden sich 2 ungepaarte Elektronen gleichen Spins. Es handelt sich also um ein Biradikal. Daher sind Oxidationsreaktionen immer Radikalreaktionen.

Antioxidativ wirksame Substanzen werden in einer Reihe von Nahrungsergänzungsmitteln z.B. als "Anti-Aging"-Präparate und zur Krankheitsprävention (z.B. vor Krebs) auf dem Markt angeboten. Die enthaltenen antioxidativen Substanzen kommen auch natürlicherweise in der Nahrung vor bzw. werden sie vielen Lebensmitteln zugesetzt. Im besonderen stellen Nahrungsergänzungsmittel nach der Definition der EU-Richtlinie 2002/46/EG, in Deutschland umgesetzt in der Nahrungsergänzungsmittelverordnung (NemV), die Gruppe an Lebensmitteln dar, die die allgemeine Ernährung ergänzen. Ihre Darreichung muss in dosierter Form (Kapseln, Tabletten, etc.) erfolgen. Sie enthalten Konzentrate von Nährstoffen oder sonstigen Stoffen mit ernährungsspezifischer Wirkung allein oder in Kombination. In der antioxidativen Prävention muss eine Kombination gefunden werden aus exogenen Antioxidantien mit einer hohen antioxidativen Kapazität. Bekannte starke Antioxidantien sind z.B. Polyphenole, Terpene und Phytoöstrogene, Coenzym Q10, Glutathion, Liponsäure, synthetische und natürliche Lebensmittelzusatzstoffe mit antioxidativer Wirkung.

Die Bestimmung des antioxidativen Potential kann mittels verschiedener Methoden erfolgen: TAC-Test (totale antioxidative Capacity), FRAP-Test (Ferric Reducing/Antioxidant Power Assay), TEAC-Test (Trolox Equivalent Antioxidant Capacity Assay) und ORAC-Test (Oxygen Radical Absorbance Capacity Test).

Angesichts der Bedeutung der Antioxidantien bei der Reduzierung der Risiken von chronischen Erkrankungen wurde 2010 in den USA die totale antioxidative Kapazität (TAC) durch Ernährung und Nahrungsergänzungsmittel bei Erwachsenen untersucht. Dabei wurden Datenbanken des US-Department für Landwirtschaft, Daten zu Nahrungsergänzungsmitteln und zum Lebensmittelverzehr von 4391 US-Erwachsenen im Alter ab 19 Jahren ausgewertet. Um die Daten zur Aufnahme von einzelnen antioxidativen Verbindungen zu TAC-Werten zu konvertieren, wurde die Messung des Vitamin-C-Äquivalent (VCE) von 43 antioxidativen Nährstoffen zuvor angewendet. Die tägliche TAC lag durchschnittlich bei 503,3 mg VCE/Tag, davon ca. 75 % aufgenommen durch die Nahrung und 25 % durch Nahrungsergänzungsmittel.

Aufgrund der zunehmenden Bedeutung von oxidativen Stress und deren Einfluss auf das allgemeine Wohlbefinden war es eine Aufgabe der vorliegenden Erfindung, eine Zusammensetzung zu entwickeln und bereitzustellen, die über eine hohe und stabile antioxidative Kapazität verfügt.

Diese Aufgabe wurde mit einer Zusammensetzung mit den Merkmalen des Anspruchs 1 gelöst.

Entsprechend umfasst die vorliegend antioxidativ wirksame Zusammensetzung 30 bis 40 Masse% Aloe vera Blattgel; 7 bis 9 Masse% an Zucker oder einem Süßungsmittel; 30 bis 40 Masse% Traubensaft; 0,2 bis 0,5 Masse% Grünteeextrakt; 0,2 bis 0,4 Masse% Xanthan; 0,1 bis 0,2 Masse% einer L-Carnitinverbindung; 0,05 bis 0,09 Masse% einer Vitaminmischung aus Vitamin B1, Vitamin B9 (Folsäure), Vitamin B12 und Vitamin E; 0,05 bis 0,09 Masse% Chlorophyll; 0,05 bis 0,09 Masse% Säuerungsmittel; 0,02 bis 0,035 Masse% Konservierungsstoffe; 0,01 bis 0,03 Masse% Aroma; 0,07 bis 0,1 Masse% Coenyzm Q10; 0,003 bis 0,06 Masse% Eisen-Verbindung; 0,01 bis 0,015 Masse% einer Cholin-Verbindung; 0,007 bis 0,01 Masse% einer Selen-Verbindung, 0,007 bis 0,012 Masse% eines Resveratrol-Extraktes, und einen Rest Wasser.

Die vorliegende antioxidativ wirksame Zusammensetzung weist bevorzugt eine antioxidative Kapazität bestimmt in Form eines ORAC-Wertes von mindestens 40.000 µmol/l auf. Bevorzugt liegt der ORAC-Wert der Zusammensetzung in einem Bereich zwischen 40.000 und 80.000 µmol/l, insbesondere bei 40.000 µmol/l.

Wie erwähnt, wurde vorliegend zur Bestimmung der antioxidativen Kapazität der ORAC-Test angewendet. Der ORAC-Test basiert auf einer fluorimetrischen Messung, in der die Reaktivität der Antioxidantien aus der Probe gegenüber Peroxylradikalen gemessen wird. Der ORAC-Test wurde vorliegend nach einer Variante von Cao und Prior et al. (1999) durchgeführt. Das Methodenprinzip beruht auf durch einen Radikalstarter permanent gebildeten Radikalen. Diese Radikale reagieren mit dem Fluoreszenz-Farbstoff Fluorescin. Der Farbstoff wird durch diese radikalische Reaktion entfärbt, d.h. die Fluoreszenz nimmt ab. Die Abnahme der Fluoreszenz wird bis zum vollständigen Fluoreszenzverlust verfolgt.

Zugesetzte Antioxidantien verringern das Ausmaß der Entfärbungsreaktion. Die Fluoreszenzabnahme wird verzögert. Das Ausmaß der Verzögerung dient als Maß für die antioxidative Wirkung der Probe (Antioxidantien). Die Messung erfolgt im Fluoreszenzphotometer bei 37°C und 485nm. Der Vorteil dieser Methode besteht darin, dass sowohl hydrophile als auch lipophile Antioxidantien erfasst werden.

Antioxidative Zusammensetzungen sind aus dem Stand der Technik bekannt. So betrifft die WO 01/62265 A1 eine Zusammensetzung zur oralen Verabreichung, die eine Hydratisierung der Haut bewirkt. Die Zusammensetzung umfasst im wesentlichen Flavanole, insbesondere in Form von Substanzen des grünen Tees, Aloe Vera, Glycerol und Wasser. WO 2009/059205 A1 betrifft Hautpflegeprodukte enthaltend unter anderem Traubensaft, Aloe Vera Saft, Vitamin E, Grünteeextrakt und weitergehende Saftextrakte. Diese Zusammensetzungen enthalten jedoch kein Cholin oder Selen. WO 2013/093863 A1 wiederum betrifft Getränke umfassend unter anderem Aloe Vera Extrakt, Traubensaftextrakt, weitere Fruchtsaftextrakte, Vitamin E, weitere Antioxidantien sowie Selen; Cholin ist jedoch nicht enthalten.

Erfindungsgemäß ist inder vorliegenden Zusammensetzung ist das mindestens eine polyphenolreiche Pflanzenextrakt ausgewählt aus einer Gruppe enthaltend ein Extrakt aus Grüntee, Traubensaft enthalten. Andere polyphenolreiche Pflanzenextrakte sind Extrakte aus Johannisbeere, Cistus, Granatapfel, Mangostane, Cranberry, Zimt/Zimtrinde, Melissenblätter, Roibis, Brombeerblätter, Himbeerblätter, Schwarztee, Lindenblüten, Majoran, Oregano, Pfefferminz, Salbei, getrocknetes Basilikum, Chilipulver, Klee, Cumin, Curry, Ingwer, Senfsaat, Muskatnuß, Oregano, Paprika, Petersilie, schwarzer/roter/weisser Pfeffer, Rosmarin, Thymian, Vanille-Schoten, Thymian, schwarze Johannisbeeren, Hagebutte, Ingwer, Pecannüsse, Schokolade, Kakao, Reis, Hirse (rot, Schwarz, etc..

Polyphenole setzen sich zusammen aus Flavonoiden in Form von Flavonen, Flavanolen, Flavonolen, Anthocyanidinen, Flavanonen und nicht-flavonoiden Polyphenolen in Form von Hydroxybenzoesäuren, Stilbenen und Hydroxyzimtsäuren. Flavonoide und Stilbene weisen anfänglich den gleichen Biosyntheseweg auf: Kondensationsreaktion von Zimtsäurederivat zu Styryl-3,5,7-triketoheptansäure. Danach erfolgt ein intramolekularer Ringschluß einerseits zu Stilbenoiden und andererseits zu Flavonoiden. Entsprechend ihres Entwicklungsstadiums einer Pflanze regulieren die beteiligte Enzyme eher die Bildung von Stilbenen (bilden Resistenzen gegen Krankheiten) oder die von Flavonoiden (Pflanzenfarbstoffe).

Erfindungsgemäß wird ein Grüntee-Extrakt als polyphenolreiches pflanzliches Extrakt verwendet. Grünteeextrakt wird bevorzugt gewonnen aus einem unfermentierten Grüntee von *Camelia sinensis.* Die frischen Blätter werden nach der Ernte kurzfristig getrocknet. Oxidative und enzymatische Vorgänge sind dadurch gehemmt. Der Extrakt wird aus einem wässrigen Auszug und anschließender Sprühtrocknung hergestellt. Der Gesamtpolyphenolgehalt liegt bei ≥80% und der Catechingehalt bei ≥60%. Der Catechingehalt von Grüntee setzt sich zusammen aus Epicatechin (EC), Epicatechingallat (ECG), Epigallocatechin (EGC) und Epigallocatechingallat (EGCG). Diese phenolischen Verbindungen sind ursächlich für die außerordentlich hohe antioxidative Kapazität von Grünteeextrakt im Vergleich zu anderen Pflanzenextrakten. So wurde für Grüntee-Extrakt eine der höchsten Konzentrationen an antioxidativer Kapazität (TAC) unter bisher untersuchten Kräutertees festgestellt.

In der vorliegenden Zusammensetzung wird der Grüntee-Extrakt in einer Menge (Masse%) zwischen 0,2 und 0.5 % verwendet.

Erfindungsgemäß wird das Porphyrin Chlorophyll verwendet, das reichhaltig in grünem Gemüse vorkommt und in *in-vitro*-Untersuchungen über eine beachtliche antioxidative Aktivität verfügt.

Die verschiedenen Typen von Chlorophyll unterscheiden sich in den Seitengruppen der Porphyrine. Insofern stehen Chlorophylle unterschiedlichster Quellen zur Auswahl: Cyanobakterien, Grünalgen, Braun-, Kiesel- und Goldalgen, Rotalgen, Purpurbakterien, rüne Schwefel- und Nichtschwefelbakterien, Grünkohl, Brennessel, Petersilie, Spinat, Broccoli, grüne Bohnen, grüne Erbsen, Gurke, Kiwis, Weißkohl, Weizen- und Gerstengras. Auch von Chlorophyll aus Weizengras konnte gezeigt werden, dass die daraus isolierten Moleküle in vitro einen DNA-Schaden durch Oxidation verhindern können.

Das mindestens eine Chlorophyll wie z.B. aus Weizen- und/oder Gerstensaftpulver wird in der vorliegenden Zusammensetzung in einer Menge (Masse%) zwischen 0,01 0,05 und 0,09 %verwendet.

Erfindungsgemäß ist die Verwendung des Stilbenoids Resveratrol. Resveratrol kommt in Form von vier Derivaten vor: (E)- und (Z)-Resveratrol und deren Glucoside, die auch als (E)-bzw. (Z)-Piceid bekannt sind. Erstmals entdeckt wurde Resveratrol in den Wurzeln von Nieswurz. 1963 wurde die Verbindung erstmals aus dem Japanischen Staudenknöterich (Polygonum cuspidatum) isoliert und identifiziert. Polygonium cuspidatum erzielt den höchsten Gehalt an Resveratrol. Es handelt sich hierbei um eine Pflanze, die ursprünglich aus der traditionellen japanischen Medizin unter dem Namen Ko-jo-kon und in der indischen Ayurveda-Medizin als Darakchasava hervorging. Resveratrol kann auch aus der Schale von Weintrauben der Arten Vitis vinifera L., V. Labrusca L. und in den Blättern gewonnen werden. So enthlaten Traubensäfte zwischen 0,1 und 6,5 mg/l Resvertrol-Derivate (überwiegend (E)- und (Z)-Piceide).

Pflanzen synthetisieren Resveratrol über p-Cumaroy-Coenzym CoA und Malonyl-Coenzym CoA, die oft unter Stressbedingungen als Schutzmechanismus gegen Pilz-, Bakterien- und Virusinfektionen induziert werden. Resveratrol besitzt neben der antioxidativen Wirkung tumorhemmende, antirhombische und phytoöstrogene Eigenschaften. Es verhindert u.a. das Zusammenkleben von Thrombozyten. Die Wirksamkeit von Resveratrol dient auch als mögliche Erklärung für das »Französische Paradoxon«. Es wird hier ein Phänomen beschrieben, dass Franzosen trotzt hohem Alkoholgenusses länger lebten als andere Bevölkerungsgruppen (z.B. Deutsche oder Amerikaner) und auch die Alterungserscheinungen weniger ausgeprägt seien. Zudem wiesen Wissenschaftler für Resvertrol ausgeprägte antimutagene Eigenschaften nach. So induziert es Phase IIdrogenmetabolisierende Enzyme und hemmt die Funktion der Enzyme Cyclooxygenase und Hydroperoxidase, die gefährliche freie Radikale freisetzen können.

In einer weiteren Variante der vorliegenden Zusammensetzung wird das mindestens eine zusätzliche Stilbenoid in Form eines Pflanzenextraktes ausgewählt aus einem Extrakt von Japanischen Staudenknöterich, Traubensaft, Traubenkern, Himbeeren, Maulbeeren, Pflaumen, Erdnüssen verwendet. Als weitere Pflanzenextrakte sind hier Rotweinextrakte, Extrakte aus Weinreben, Kakaobohnen, Heidel-, Blau- und Preisselbeeren, Cranberries, Extrakte aus Jackfrucht, Rhabarber, Hopfen, Eukalyptus, verschiedene Liliengewächse, Hülsenfrüchte etc. einsetzbar. Bevorzugt ist dabei die Verwendung eines Pflanzenextraktes aus Knöterich und/oder aus Traubensaft, insbesondere weißen Traubensaft.

Der Pflanzenextrakt aus Knöterich z.B. eine 10% Resveratol-haltiger Knöterichextrakt kann in einer Menge (Masse%) zwischen 0,007 und 0,012% in der vorliegenden Zusammensetzung zum Einsatz kommen.

Traubensaft, insbesondere weißem Traubensaft wird erfindungsgemäß in einer Menge (Masse%) zwischen 30 und 40% in der vorliegenden Zusammensetzung verwendet.

Erfindungsgemäß enthält die vorliegende Zusammensetzung mindestens ein Vitamin ausgewählt aus der Gruppe enthaltend Vitamin B1, Vitamin B9 (Folsäure), Vitamin B12 und Vitamin E.

Vitamin B12 wird auch als Cobalamin bezeichnet. Es besteht aus einem Ringsystems aus 4 Pyrrolringen. Das Zentralatom dieses komplexen Ringsystems ist ein Cobaltatom. Vitamin B12 dient zur Wiederherstellung der aktiven Form der Folsäure als Methylfolat. Methylfolat ist ein Reaktionsprodukt bei der Methylierung von Homocystein zu Methionin im Zusammenhang mit der DNA-Synthese. Diese Funktion erklärt die Bedeutung von Vitamin B12 für sich schnell teilendes Zellgewebe, wie z.B. die Zellen des Knochenmarkes.

Erfindungsgemäß wird eine Mischung aus Vitamin B1, Vitamin B9 (Folsäure), Vitamin B12 und Vitamin E verwendet, wobei diese Vitaminmischung in einer Menge zwischen 0,05 und 0,09% in der Zusammensetzung vorliegt.

Erfindungsgemäß wird der vorliegenden Zusammensetzung L-Carnithin oder mindestens ein Salz davon, bevorzugt L-Carnithintartat, zugegeben. L-Carnithin ist als quarternäre Ammoniumverbindung von Natur aus ein Bestandteil der gestreiften Muskulatur und der Leber. Es handelt sich um eine Transportsubstanz für Acetylgruppen vorwiegend zwischen dem zytoplasmatischen Raum der Zelle und dem intramitochoindrialen Raum. Auf diese Weise können längerkettige Fettsäuren in die Mitochondrien eingeschleust werden. L-Carnithin ist daher unverzichtbar für die Fettverbrennung, verbessert den Energiehaushalt, erhöht die sportliche Ausdauer und körperliche Leistungsfähigkeit.

Es ist auch wünschenswert, der vorliegenden Zusammensetzung Spurenelemente ausgewählt aus einer Gruppe enthaltend Selen (Se), Chrom (Cr), Cobalt (Co), Eisen (Fe), lod (I), Kupfer (Cu), Mangan (Mn), Molybdän (Mo) und Zink (Zn) zuzufügen.

Erfindungsgemäß wird Selen, z.B. in Form eines Salzes wie Natriumselenit zugegeben. Selen ist z.B. in Glutathionperoxidase und Dejodase enthalten. Man bezeichnet Selen auch als Zellschutzstoff, der die Erbanlangen vor Veränderungen schützt. Es verhindert die Lipidperoxidation in Membranen von Zellen und verhindert somit die Bildung zelltoxischer Verbindungen vermutlich durch Inhibition durch einen Radikal-Abbruch-Mechanismus. Es handelt sich um ein typisches Antioxidans. Selen kann auch in Form von Natriumselenat, Natriumhydrogenselenit und/oder Selenhefe eingesetzt werden.

Die in der vorliegenden Zusammensetzung verwendete Menge an Selen, insbesondere Natriumselenit beträgt in Masse% zwischen 0,007 und 0,01 %.

Des Weiteren weist die vorliegende Zusammensetzung Cholin, bevorzugt als Cholinbitartrat oder Cholinchlorid, auf.

Cholin ist ein biologisch wichtiges biogenes Amin. Durch das Enzym Cholinacetyltransferase wird aus Cholin und aktivierter Essigsäure der Überträgerstoff Acetylcholin biosynthetisiert. Als Bestandteil des Lecithins ist Cholin Baustein aller tierischen und pflanzlichen Zellen, vor allem biologischer Membranen. Der Bedarf bei übernormalen physischen Belastungen (z.B. Leistungssport) kann nicht völlig über die Nahrungsaufnahme und durch Eigensynthese des Organismus gedeckt werden.

Die Menge an verwendeten Cholin in der vorliegenden Zusammensetzung beträgt zwischen 0,01 und 0,015 %.

Auch kann in der vorliegenden Zusammensetzung mindestens ein Chinon-Derivat, insbesondere aus der Gruppe der Ubichinone, Phyllochinone und/oder Pyrrolochinolinchinon PQQ vorliegen.

Erfindungsgemäß wird Coenzym Q10 verwendet. Coenzym Q10 ist ein Ubichinon. Diese Wirkstoffgruppe ist auf einem Grundgerüst aus 2,3-Dimethoxy-5-methyl-1 ,4-benzochinon mit einer isoprenoiden Seitenkette unterschiedlicher Länge aus 6-10 Dehydroisopren-Einheiten aufgebaut. Coenzym Q10 weist 10 Isopreneinheiten auf. Coenzym Q10 wird vom menschlichen Organismus aus Phenylalanin synthetisiert. Beispielsweise bei stark erhöhter Radikalbelastung und damit gesteigerter Lipidperoxidation reicht die körpereigene Synthese von Coenzym Q10 nicht aus und sollte daher supplementiert werden.

Die in der vorliegenden Zusammensetzung verwendete Menge an Coenzym Q10 beträgt zwischen kann in Masse% zwischen 0,07 und 0,1%,.

Als weiteren Bestandteil kann die vorliegende Zusammensetzung mindestens einen Extrakt aus der Pflanze der Stevien (Stevia) aufweisen. Das Verbreitungsgebiet der Stevia reicht von den westlichen USA über Zentralamerika bis nach Paraguay und Brasilien in Südamerika.

Aus der Stevia wird ein pflanzliches Süßungsmittel gewonnen, dessen Süßkraft auf Steviosiden beruht.

Das mindestens eine Stevia-Extrakt kann aus einer der folgenden Stevia Pflanzen gewonnen werden: *Stevia amambayensis, Stevia ammotropha, Stevia amplexicaulis, Stevia apensis, Stevia aristata, Stevia balansae, Stevia breviaristata, Stevia catharinensis, Stevia commixta, Stevia cuneata, Stevia entreriensis, Stevia estrellensis, Stevia eupatoria, Stevia lemmonii, Stevia leptophylla, Stevia micrantha, Stevia ovata, Stevia parvifolia, Stevia plummerae, Stevia rebaudiana, Stevia rojasii, Stevia sabulonis, Stevia salicifolia, Stevia satureiifolia, Stevia selloi, Stevia serrata, Stevia spathulata, Stevia veronicae, Stevia villaricensis* und/oder *Stevia viscida.*

Die Menge an zu der vorliegenden Zusammensetzung zugegeben Stevia-Extrakt kann (in Masse%) zwischen 0,01 und 0,1 %, bevorzugt zwischen 0,01 und 0,05 %, insbesondere bevorzugt zwischen 0,01 und 0,03 %, ganz besonders bevorzugt 0,2% betragen.

Als weiteren Inhaltsstoff enthält die vorliegende Zusammensetzung mindestens ein Süßungsmittel oder Zucker. Bevorzugterweise weist die Zusammensetzung ein Monosaccharid, bevorzugt Pentosen oder Hexosen, insbesondere Ribose, Arabinose, Xylose, Glucose, Mannose, Galactose, Fructose, besonders bevorzugt Glucose, Glacatose, Fructose auf.

Monosaccharide wie Glucose (auch als Dextrose oder Traubenzucker bezeichnet) können vom menschlichen Organismus ohne vorherige Spaltung sofort resorbieren werden. Zellen des Gehirns und die Erythrozyten müssen ihren Energie-Bedarf im Gegensatz zu allen anderen Zellen des Organismus vorwiegend durch Glucose decken. Glucose dient als Nähr- und Kräftigungsmittel einerseits und bildet das Ausgangsprodukt für Diabetiker-Süssungsmittel.

Die Menge an zugesetzter Glucose beträgt in der vorliegenden Zusammensetzung (in Masse%) zwischen 7 und 9%.

Auch wird erfindungsgemäß der vorliegenden Zusammensetzung Aloe vera- zuzugeben. Die Menge an Aloe vera-, das z.B. in Form eines Aloe vera Gels eingesetzt werden kann, liegt in Masse% zwischen 30 und 40%.

Auch ist die Verwendung von mindestens einer Eisenhaltigen Verbindung in der vorliegenden Zusammensetzung von Vorteil. So kann z.B. Eisen(III)-pyrophosphat der Zusammensetzung zugeführt werden. Ebenfalls können Eisencarbonat, Eisencitrat, Eisenammoniumcitrat, Eisengluconat, Eisenfumarat, Eisennatriumdiphosphat, Eisenlactat, Eisensulfat, Eisendiphosphat, Eisensaccharat, elementares Eisen (Carbonyl + elektrolytisch + wasserstoffreduziert) und/oder Eisen-Bisglycinat als Eisen-Quelle verwendet werden. Die Vorteile der Einnahme von Eisen sind vielfältig für den Menschen. So trägt Eisen zur Verringerung von Müdigkeit und Ermüdung, zu einer normalen kognitiven Funktion, zu einem normalen Energiestoffwechsel, zur normalen Bildung von roten Blutkörperchen und Hämoglobin, zu einem normalen Sauerstofftransport im Körper sowie zu einer normalen Funktion des Immunsystems bei. Darüberhinaus weist Eisen eine Funktion bei der Zellteilung auf.

Die Menge an Eisen, insbesondere an Eisen(III)-pyrophosphat, beträgt in der vorliegenden Zusammensetzung in Masse% zwischen 0,03 und 0,06%, ganz besonders bevorzugt 0,05% betragen. Der Eisenbedarf für Männer beträgt üblicherweise 10mg/Tag, für Frauen 15mg/Tag und kann sich bei Schwangeren und Stillenden Müttern auf 30mg/Tag erhöhen.

Neben den bisher angeführten Inhaltsstoffen kann der vorliegenden Zusammensetzung zusätzlich mindestens ein Säuerungsmittel, bevorzugt Citronensäure, mindestens einen Konservierungsstoff, insbesondere Kaliumsorbat und Natriumbenzoat, mindestens ein Stabilisator, bevorzugt Xanthan, natürliche Aromen sowie mindestens ein weiteres Antioxidationsmittel, bevorzugt Ascorbinsäure (Vitamin C) zugegeben werden.

Die vorliegende Zusammensetzung ist als Mittel zur Behandlung und/oder Prophylaxe von stressbedingten Erkrankungen einsetzbar. So führt die vorliegende Zusammensetzung zur Erhöhung der Leistungsfähigkeit, zur Verringerung von Müdigkeit und Ermüdung, zum Abbau von oxidativem Stress, dient der Prävention von Herz-/Kreislauferkrankungen, Atheriosklerose, Diabetis, Augenerkrankungen, Demenzen, Krebs und entzündliche Erkrankungen wie z.B. Rheuma, Arthrosen, entzündliche Darmerkrankungen.

Ganz besonders dient die vorliegende Zusammensetzung der Reduktion von reaktiven Sauerstoffspezien (ROS), insbesondere im Menschen, und somit der Vorbeugung von Erkrankungen, die durch ROS hervorgerufen werden können, und der Verlangsamung des allgemeinen Alterungsprozesse im Menschen.

Ebenfalls ist die vorliegende Zusammensetzung als Mittel zur Steigerung der mentalen Leistungsfähigkeit einsetzbar.

Die Erfindung wird im Folgenden ausführlicher anhand von Ausführungsbeispielen erläutert.

### Ausführungsbeispiel 1

Eine bevorzugte Ausführungsform der erfindungsgemäßen Zusammensetzung weist die in Tabelle 1 angeführten Inhaltsstoffe auf.

**Tabelle 1: Inhaltsstoffe einer Ausführungsform der erfindungsgemäßen Zusammensetzung**

| **Chargengröße 100kg** | | |
|---|---|---|
| **Pos.** | **Rohstoff** - **Bezeichnung** | **Menge [Kg]** |
| 1 | Aloe Vera Blattgel | 36,000 |
| 2 | Traubensaftkonzentrat | 32,500 |
| 3 | Wasser | 21,600 |
| 4 | Zucker / Süßungsmittel | 8,52 |
| 5 | Grünteeextrakt | 0,400 |
| 6 | Xanthan | 0,300 |
| 7 | L-Carnitinverbindung | 0,180 |
| 8 | Vitaminmischung aus Vitamin B1, B12, E, Folsäure | 0,0842 |
| 9 | Chlorophyll | 0,0800 |
| 10 | Säuerungsmittel | 0,096 |
| 11 | Konservierungsstoffe | 0,034 |
| | | |
| 12 | Aroma | 0,020 |
| | | |
| 13 | Coenzym Q10 | 0,0904 |
| 14 | Eisen-Verbindung | 0,0506 |
| | | |
| 15 | Cholinhyverbindung | 0,0121 |
| 16 | Selenverbindung | 0,00937 |
| 17 | Resveratrolextrakt | 0,0100 |

### Ausführungsbeispiel 2

Die Bestimmung des ORAC-Wertes einer Zusammensetzung gemäß Ausführungsbeispiel 2 wurde in folgender Weise durchgeführt.

Das der ORAC Messung zugrunde liegende Messprinzip basiert darauf, dass freie Radikale (vorliegend 2,2'-Azobis(2-Amidinopropan)dihydrochlorid, AAPH) ein fluoreszierendes Molekül (vorliegend Fluorescein) zersetzen und somit die Gesamtfluoreszenz einer Probe reduzieren. Diese Änderung dient zur Darstellung der Schädigung durch freie Radikale. Durch die Zugabe von Antioxidantien wird die Schädigung gehemmt, die Fluoreszenz bleibt erhalten. Das Vitamin E-Derivat Trolox dient als Referenz, weswegen das Ergbenis in in Trolox-Äquivalenten angegeben wird.

Zur Probenvorbereitung wird zunächst 1 mL einer Probe in 2,5 mL Aceton-Wassergemisch (50:50, v/v) gelöst und mit dest. Wasser auf 10 mL aufgefüllt (Verdünnung 1:10).

Eine Fluorescein-Arbeitslösung wird durch Verdünnen einer Stammlösung (c=4,5*10-3 M, 1:200,000) hergestellt. Ein Trolox-Standard (150 µM, 75 µM, 30 µM, 15 µM) wird bereitgestellt. Die Messproben werden gemischt, in 96-Mikrotiterplatten übertragen und 5 min bei 37°C inkubiert. Anschließend erfolgt die Zugabe von AAPH mit sofortiger Messung der Fluoreszenz bei 485 nm (80 mal je 1,5 min).

Die durch die kinetische Messung gewonnenen Ergebnisse werden in die entsprechenden Trolox-Äquivalente (TE) umgerechnet.

### Ausführungsbeispiel 3

Zur Untersuchung des antioxidativen Effektes der Zusammensetzung des Ausführungsbeispiels 1 wurde ein Anwendertest durchgeführt.

Das Erfordernis einer Antioxidantien-Supplementierung in der Prävention kann über ausgewählte und vom Grad des oxidativen Stresses abhängigen Marker in Urin oder im Blut/Serum/Plasma bestimmt werden. Daher wurden im Anwendertest die Auswahl der Probanden anhand von Kreatinin und Isoprostane aus Urin und/oder Lipidperoxide aus dem Blut getroffen.

Mittels der vorliegenden Studie wurde untersucht, ob und in welchem Ausmaß zusätzlich zur normalen Ernährung zugeführte Antioxidantien in Form der vorliegenden Zusammensetzung mit einem hohem ORAC-Wert Einfluss auf oxidative Stressmarker in Blut und Urin von gesunden, erwachsenen Probanden nehmen, die erhöhte oxidative Stressmarker im Blut Lipidperoxidase bzw. Urin (Isoprostane) aufweisen. Auch sollte nachgewiesen werden, welche Rolle die Höhe der Ausgangswerte oxidativer Stressmarker auf den Einfluss zusätzlicher Antioxidantien spielt und gleichzeitig sollte der Konzentrationsverlauf der Mikronährstoffe Selen, Co-Enzym Q 10 und Vitamin B 12 im Blut kontrolliert werden, die ebenfalls über die vorliegende Zusammensetzung zugeführt werden.

Es handelt sich um eine monozentrische, prospektive, Placebo-kontrollierte Beobachtungsstudie über drei Wochen zum Einfluss eines täglich eingenommenen antioxidativen, mikronährstoffreichen Getränks mit einem durchschnittlichen ORAC-Wert von 40 000 µMol TE/l auf die oxidativen Stressmarker: Isoprostane und Kreatinin im Urin, Lipidperoxidase im Blut sowie die Mikronährstoffe Selen, Co-Enzym Q 10 und Vitamin B 12 im Blut von gesunden Erwachsenen mit erhöhten oxidativen Stressparametern.

Die Untersuchung wurde an einer Gruppe von 20 Personen beiderlei Geschlechts durchgeführt, von denen randomisiert 15 dem Verumarm (echte Behandlung mit tatsächlich vorhandenem "Wirkstoff" im Gegensatz vom Placebo) und 5 der Placebogruppe zugeteilt werden.

Es wurden erwachsene Personen im Alter zwischen 25 und 50 Jahren, mit einem BMI < 35, ohne schwerwiegende Vorerkrankungen mit den folgenden oxidativen Stressparametern ausgewählt: Blut: Lipidperoxide > 275 µMol/l und/oder Urin: 8-Epi-Prostaglandin > 3,5 µg/g Kreatinin. Probanden mit ASA Klasse > 2 (z.B. insulinpflichtiger Diabetes, maligne Erkrankungen, schwere Nieren- oder Leberfunktionsstörungen) wurden vom Test ausgeschlossen.

Folgende primäre Messwerte wurden ermittelt:
- Isoprostantest im Urin (8-Epi-Prostaglandin) zur Bestimmung der Belastung mit reaktiven Sauerstoffverbindungen (freien Radikalen)
- Kreatinin im Urin als Maß für die Konzentrationsleistung der Niere und Marker für die NO-Bildung (nitrosativer Stress)
- Lipidperoxide im Blut zur Bewertung einer oxidativen Belastung (Lipidperoxidation)
- Selen, Co-Enzym Q 10, Vitamin B 12 im Blut.

Außerdem werden demographische Faktoren (Alter, Geschlecht, Geburtsdatum, Größe in cm und Körpergewicht in kg) erfasst.

Die Probanden nahmen täglich die vorliegende Zusammensetzung gemäß Verzehrempfehlung (einmalig 80ml/Tag entspricht 3.200 µMol TE/Tag) ein.

Im Ergebnis führt die 3-wöchige Einnahme eines antioxidativen Getränks mit hohem ORAC-Wert (40000 µMol TE/l) zu einer Verminderung oxidativer Stressmarker in Blut (Lipidperoxidase) und Urin (Isoprostane, Kreatinin) bei gesunden Erwachsenen.

## Patentansprüche

1. Antioxidativ wirksame Zusammensetzung **gekennzeichnet durch**
- 30 bis 40 Masse% Aloe vera Blattgel;
- 7 bis 9 Masse% eines Zuckers oder eines Süßungsmittels;
- 30 bis 40 Masse% Traubensaft;
- 0,2 bis 0,5 Masse% Grünteeextrakt;
- 0,2 bis 0,4 Masse% Xanthan;
- 0,1 bis 0,2 Masse% einer L-Carnitinverbindung;
- 0,05 bis 0,09 Masse% einer Vitaminmischung aus Vitamin B1, Vitamin B9 (Folsäure), Vitamin B12 und Vitamin E;
- 0,05 bis 0,09 Masse% Chlorophyll;
- 0,05 bis 0,09 Masse% eines Säuerungsmittels;
- 0,02 bis 0,035 Masse% Konservierungsstoffe;
- 0,01 bis 0,03 Masse% Aroma;
- 0,07 bis 0,1 Masse% Coenyzm Q10;
- 0,003 bis 0,06 Masse% einer Eisen-Verbindung;
- 0,01 bis 0,015 Masse% einer Cholin-Verbindung;
- 0,007 bis 0,01 Masse% einer Selen-Verbindung,
- 0,007 bis 0,012 Masse% eines Resveratrol-Extraktes, und
- Rest Wasser.

2. Zusammensetzung nach Anspruch 1, **gekennzeichnet durch** einen ORAC-Wert zwischen 40.000 und 80.000 µmol/l.

3. Zusammensetzung nach einem der vorhergehenden Ansprüche, **dadurch gekennzeichnet, dass** das mindestens eine Resveratrol Extrakt in Form eines Pflanzenextraktes ausgewählt ist aus einem Extrakt von Japanischen Staudenknöterich.

4. Zusammensetzung nach einem der vorhergehenden Ansprüche **dadurch gekennzeichnet, dass** die Zusammensetzung eine Cholin-Verbindung in Form von Cholinbitartrat oder Cholinchlorid aufweist.

5. Zusammensetzung nach einem der vorhergehenden Ansprüche **gekennzeichnet durch** mindestens die Zugabe von mindestens einem Stevia-Extrakt als Süßungsmittel.

6. Zusammensetzung nach einem der vorhergehenden Ansprüche **gekennzeichnet durch** Zugabe vom mindestens einem Monosaccharid als Zucker, bevorzugt Pentosen oder Hexosen insbesondere Ribose, Arabinose, Xylose, Glucose, Mannose, Galactose, Fructose, besonders bevorzugt Glucose, Galactose, Fructose.

7. Zusammensetzung nach einem der vorhergehenden Ansprüche **gekennzeichnet durch** die Zugabe von mindestens Eisen(III)-pyrophosphat als Eisenverbindung.

8. Zusammensetzung nach einem der vorhergehenden Ansprüche als Mittel zur Verwendung in der Behandlung und/oder Prophylaxe von stressbedingten Erkrankungen.

9. Zusammensetzung nach einem der Ansprüche 1 bis 7 als Mittel zur Verwendung zur Steigerung der mentalen Leistungsfähigkeit.

10. Zusammensetzung nach einem der Ansprüche 1 bis 7 als Mittel zur Verwendung zur Reduktion von reaktiven Sauerstoffspezien (ROS), insbesondere im Menschen.

## Claims

1. Antioxidant composition **characterized by**
- 30 to 40 mass% Aloe vera leaf gel;
- 7 to 9 mass% of a sugar or a sweetener;
- 30 to 40 mass% grape juice;
- 0.2 to 0.5 mass% green tea extract;
- 0.2 to 0.4 mass% xanthan gum;
- 0.1 to 0.2 mass% of an L-carnitine compound;
- 0.05 to 0.09 mass% of a vitamin mixture of vitamin B1, vitamin B9 (folic acid), vitamin B12 and vitamin E;
- 0.05 to 0.09 mass% chlorophyll;
- 0.05 to 0.09 mass% of an acidifying agent;
- 0.02 to 0.035 mass% preservatives;
- 0.01 to 0.03 mass% aroma;
- 0.07 to 0.1 mass% Coenyzm Q10;
- 0.003 to 0.06 mass% of an iron compound;
- 0.01 to 0.015 mass% of a choline compound;
- 0.007 to 0.01 mass% of a selenium compound,
- 0.007 to 0.012 mass% of an extract of resveratrol, and
- Residual water.

2. Composition according to claim 1, **characterized by** an ORAC value between 40,000 and 80,000 µmol/l.

3. Composition according to any one of the preceding claims, **characterized in that** the at least one resveratrol extract is in the form of a plant extract selected from an extract of Japanese knotweed.

4. Composition according to any one of the preceding claims **characterized in that** the composition comprises a choline compound in the form of choline bitartrate or choline chloride.

5. Composition according to any one of the preceding claims **characterized by** at least the addition of at least one stevia extract as sweetener.

6. Composition according to any one of the preceding claims **characterized by** addition of at least one monosaccharide as sugar, preferably pentoses or hexoses in particular ribose, arabinose, xylose, glucose, mannose, galactose, fructose, particularly preferably glucose, galactose, fructose.

7. Composition according to any one of the preceding claims **characterized by** the addition of at least ferric pyrophosphate as an iron compound.

8. Composition according to any one of the preceding claims as an agent for use in the treatment and/or prophylaxis of stress-related diseases.

9. Composition of any one of claims 1 to 7 as an agent for use in enhancing mental performance.

10. Composition of any one of claims 1 to 7 as an agent for use in reducing reactive oxygen species (ROS), particularly in humans.

## Revendications

1. Composition à action antioxydante **caractérisée par**
- 30 à 40% en masse de gel de feuilles d'aloe vera ;
- 7 à 9% en masse d'un sucre ou d'un édulcorant ;
- 30 à 40% en masse de jus de raisin ;
- 0,2 à 0,5 % en masse d'extrait de thé vert ;
- 0,2 à 0,4 % en masse de xanthane ;
- 0,1 à 0,2 % en masse d'un composé de L-carnitine ;
- 0,05 à 0,09% en masse d'un mélange de vitamines composé de vitamine B1, vitamine B9 (acide folique), vitamine B12 et vitamine E ;
- 0,05 à 0,09 % en masse de chlorophylle ;
- 0,05 à 0,09 % en masse d'un acidifiant ;
- 0,02 à 0,035 % en masse de conservateurs ;
- 0,01 à 0,03 % en masse d'arôme ;
- 0,07 à 0,1 % en masse de coenyzme Q10 ;
- 0,003 à 0,06 % en masse d'un composé de fer ;
- 0,01 à 0,015 % en masse d'un composé de choline ;
- 0,007 à 0,01 % en masse d'un composé de sélénium ;
- 0,007 à 0,012 % en masse d'un extrait de resvératrol, et
- le reste étant de l'eau.

2. Composition selon la revendication 1, **caractérisée par** une valeur ORAC comprise entre 40.000 et 80.000 µmol/l.

3. Composition selon l'une quelconque des revendications précédentes, **caractérisée en ce que** ledit au moins un extrait de resvératrol sous forme d'un extrait végétal est choisi parmi un extrait de Renouée du Japon.

4. Composition selon l'une quelconque des revendications précédentes, **caractérisée en ce que** la composition comprend un composé de choline sous forme de bitartrate de choline ou de chlorure de choline.

5. Composition selon l'une quelconque des revendications précédentes, **caractérisée par** au moins l'addition d'au moins un extrait de stévia comme édulcorant.

6. Composition selon l'une quelconque des revendications précédentes, **caractérisée par** l'addition d'au moins un monosaccharide sous forme de sucre, de préférence des pentoses ou des hexoses, en particulier du ribose, de l'arabinose, du xylose, du glucose, du mannose, du galactose, du fructose, de manière particulièrement préférée du glucose, du galactose, du fructose.

7. Composition selon l'une quelconque des revendications précédentes, **caractérisée par** l'addition d'au moins du pyrophosphate ferrique en tant que composé de fer.

8. Composition selon l'une quelconque des revendications précédentes, en tant qu'agent à utiliser dans le traitement et/ou la prophylaxie de maladies liées au stress.

9. Composition selon l'une quelconque des revendications 1 à 7, en tant qu'agent destiné à utiliser pour améliorer la performance mentale.

10. Composition selon l'une quelconque des revendications 1 à 7, en tant qu'agent à utiliser pour réduire des espèces réactives de l'oxygène (ROS), en particulier chez l'homme.
